# EUROPEAN PATENT APPLICATION

(11) **EP 3 159 116 A1**
(43) Date of publication of application: **26.04.2017**
(21) Application number: 15810031.3
(22) Date of filing: 26.05.2015
(51) Int. Cl.: B25J 3/00

(54) **MEDICAL TREATMENT SYSTEM SETTING METHOD**

(30) Priority: 17.06.2014 JP 2014124524
(71) Applicant: Olympus Corporation, Hachioji-shi, Tokyo 192-8507 (JP)
(72) Inventor: KOMURO, Takahiro, Tokyo 192-8507 (JP); IIDA, Masatoshi, Tokyo 192-8507 (JP)
(74) Representative: Gunzelmann, Rainer
(86) International application number: PCT/JP2015/065139
(87) International publication number: WO 2015/194333

(57) **Abstract**

A method of setting a medical system, in which at least one treatment tool and at least one manipulation input unit for manipulating the treatment tool are connectable to each other, includes a step of acquiring first information capable of identifying the treatment tool and second information capable of identifying the manipulation input unit, a step of associating the treatment tool with the manipulation input unit on the basis of a comparison between the first information and the second information, and a step of associating an input to the manipulation input unit with an operation of the treatment tool with respect to the manipulation input unit associated with the treatment tool on the basis of the comparison between the first information and the second information.

## Description

### Technical Field

The present invention relates to a method of setting a medical system.

Priority is claimed on Japanese Patent Application No. 2014-124524, filed June 17, 2014, the content of which is incorporated herein by reference.

### Background Art

Conventionally, a medical system including a treatment tool manipulated by a manipulation unit is known (see, for example, Patent Literatures 1 and 2).

In the medical systems, a memory in which a type of treatment tool or various parameters are stored is mounted on the treatment tool, and the type of treatment tool can be recognized or parameters can be updated.

### Citation List

### Patent Literature

Patent Literature 1: United States Patent No. 7048745
Patent Literature 2: United States Patent No. 7239940

### Summary of Invention

### Technical Problem

In a medical system in which a plurality of treatment tools and a plurality of manipulation input units can be connected, it is necessary to perform association of the treatment tool with the manipulation input unit when the treatment tool or the manipulation input unit begins to be used. Manual association of the plurality of treatment tools with the plurality of manipulation input units is complicated, and a simple and easy setting method is required.

The present invention has been made in view of the above circumstances, and an object thereof is to provide a method of setting a medical system in which a treatment tool and a manipulation input unit are simply and easily associated with each other.

### Solution to Problem

According to a first aspect of the present invention, a method of setting a medical system, in which at least one treatment tool and at least one manipulation input unit for manipulating the treatment tool are connectable to each other, includes: a step of acquiring first information capable of identifying the treatment tool and second information capable of identifying the manipulation input unit; a step of associating the treatment tool with the manipulation input unit on the basis of a comparison between the first information and the second information; and a step of associating an input to the manipulation input unit with an operation of the treatment tool with respect to the manipulation input unit associated with the treatment tool on the basis of the comparison between the first information and the second information.

According to a second aspect of the present invention, in the method of setting the medical system according to the first aspect, the first information may be acquired from image information displayed on the treatment tool.

According to a third aspect of the present invention, in the method of setting the medical system according to the first aspect, the second information may be acquired from a storage element mounted on the manipulation input unit.

According to a fourth aspect of the present invention, in the method of setting the medical system according to the first aspect, third information indicating a correspondence relationship between the input to the manipulation input unit and the operation of the treatment tool may be acquired from a storage element mounted on the manipulation input unit.

According to a fifth aspect of the present invention, in the method of setting the medical system according to the fourth aspect, a plurality of operation elements in the treatment tool may be individually settable for a plurality of input units in the manipulation input unit.

### Advantageous Effects of Invention

According to each aspect, it is possible to provide the method of setting a medical system in which the treatment tool and the manipulation input unit are simply and easily associated with each other.

### Brief Description of Drawings

Fig. 1 is an entire view of a medical system according to an embodiment of the present invention.
Fig. 2 is a schematic diagram of the medical system.
Fig. 3 is a schematic diagram of a control unit of the medical system.
Fig. 4A is a schematic diagram of first information of the medical system.
Fig. 4B is a schematic diagram of second information of the medical system.
Fig. 4C is a schematic diagram of third information of the medical system.
Fig. 5 is a flowchart illustrating a method of setting the medical system.
Fig. 6 is a flowchart illustrating the method of setting the medical system.
Fig. 7 is a flowchart illustrating the method of setting the medical system.
Fig. 8 is a flowchart illustrating the method of setting the medical system.
Fig. 9 is a flowchart illustrating the method of setting the medical system.
Fig. 10 is a flowchart illustrating the method of setting the medical system.
Fig. 11 is a flowchart illustrating the method of setting the medical system.

### Description of Embodiments

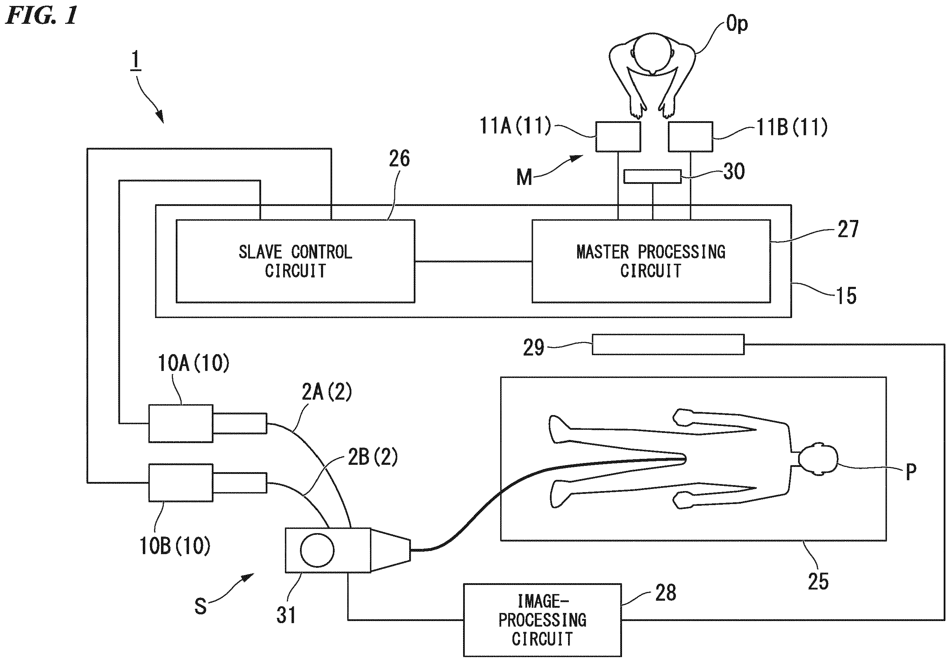

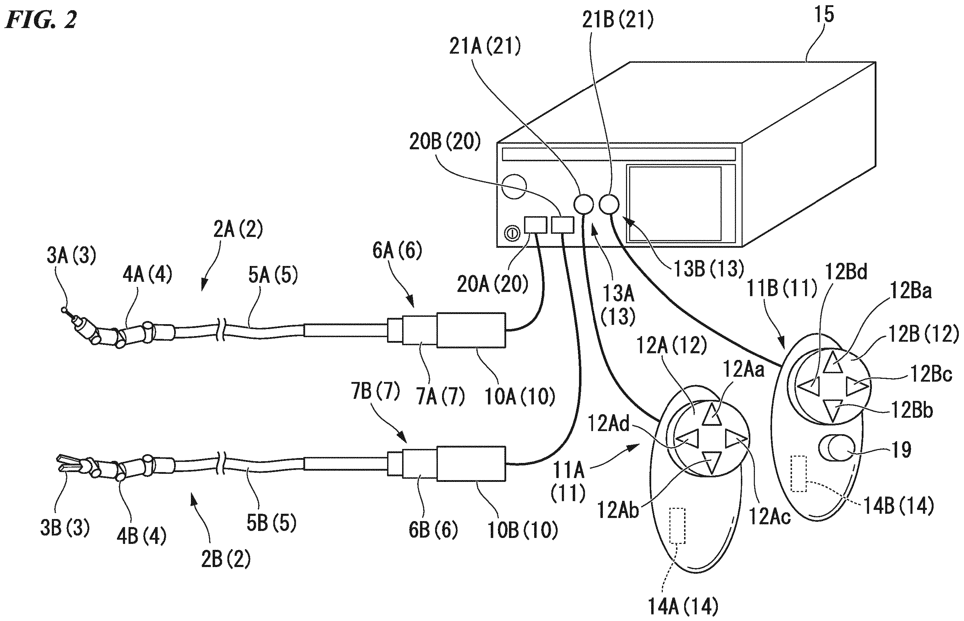

An embodiment of the present invention will be described. Fig. 1 is an entire view of a medical system according to this embodiment. Fig. 2 is a schematic diagram of the medical system.

The medical system 1 according to this embodiment is applicable to a medical manipulator system for performing surgical treatment on a patient.

Fig. 1 illustrates an example of a medical manipulator system of a master-slave type into which the medical system 1 according to this embodiment is incorporated. The medical manipulator system of a master-slave type is a system including a master M that is an input unit that receives a manipulation input from a doctor (operator) and a slave S that is a treatment portion that perform a treatment on a patient, in which the slave S is remotely controlled to follow a manipulation input of the master M. In this embodiment, an endoscope 31 and a treatment tool 2 correspond to the slave S, and manipulation input units 11A and 11B and a setting manipulation unit 30 correspond to the master M.

The medical manipulator system illustrated in Fig. 1 includes a surgical table 25, the endoscope 31, the treatment tool 2, a slave control circuit 26, a manipulation input unit 11, a master processing circuit 27, an image-processing circuit 28, and a display 29.

The surgical table 25 is a table on which a patient P serving as an observation and treatment target is placed. The endoscope 31 and the treatment tool 2 are installed near the surgical table 25. The endoscope 31 and the treatment tool 2 may be installed at the surgical table 25.

Each treatment tool 2 includes a plurality of multi-degree-of-freedom joints. The treatment tool 2 performs treatment on the patient P placed on the surgical table 25 by bending each multi-degree-of-freedom joint. The respective multi-degree-of-freedom joints are individually driven by a driving unit 10. As the driver 10, for example, a motor having a servo mechanism including an incremental encoder, a decelerator, or the like may be used. Operation control of the driving unit 10 is performed by the slave control circuit 26.

The treatment tool 2 may be rigid or may be flexible. That is, as the treatment tool 2, a treatment tool in which an actuating body for performing a treatment on a living body is operated by pushing and pulling a rigid rod, or a treatment tool in which an actuating body for performing a treatment on a living body is operated by pulling a flexible wire can be appropriately selected and adopted. Even when the treatment tool 2 is rigid, the treatment tool 2 may have a configuration in which the actuating body is operated by pulling the flexibly wire. In this embodiment, the treatment tool 2 has a configuration in which a driving force for operating an actuating body is delivered to an actuating body through a flexibly wire.

In Fig. 1, for example, the treatment tool 2 is flexible, is inserted into, for example, a treatment tool channel (not illustrated) of the endoscope 31, and is inserted into a body of the patient P. The endoscope 31 and the treatment tool 2 inserted into the endoscope 31 are introduced from a natural opening of the patient P such as a mouth or an anus into the body via a gastrointestinal tract or the like. The treatment tool 2 may be inserted into an overture attached to the endoscope 31.

The slave control circuit 26 includes, for example, a CPU, a memory, and the like. The slave control circuit 26 stores a predetermined program for controlling the treatment tool 2. The slave control circuit 26 controls an operation of the treatment tool 2 in accordance with a control signal from the master processing circuit 27. That is, the slave control circuit 26 specifies the treatment tool 2 that is a manipulation target of the master M manipulated by an operator Op on the basis of a control signal from the master processing circuit 27, and calculates a driving amount necessary for causing the specifies treatment tool 2 to perform a movement corresponding to the master manipulation amount of the operator Op.

The slave control circuit 26 controls an operation of the treatment tool 2 that is a manipulation target of the manipulation input unit 11 depending on the calculated driving amount. In this case, the slave control circuit 26 inputs a driving signal to the corresponding driving unit 10, and controls a magnitude or a polarity of the driving signal such that the driving amount of the treatment tool 2 that is the manipulation target becomes a target driving amount, depending on a detection signal that is input from a position detector of a power unit depending on the operation of the corresponding driving unit 10.

The manipulation input unit 11 includes a plurality of switches or a joystick. Pressing of the switch and a tilt angle of the joystick are detected in the master processing circuit 27 as a manipulation amount of the manipulation input unit 11. The manipulation input unit 11 may include a link mechanism. In this case, a position detector such as an incremental encoder is provided in each link constituting the link mechanism. By detecting an operation of each link using the position detector, a manipulation amount of the manipulation input unit 11 is detected in the master processing circuit 27.

The setting manipulation unit 30 includes various manipulation members, such as a clutch button for switching between control connection/disconnection of the master M to/from the slave S, a scaling changing switch for changing an operation ratio of the master M and the slave S, and a foot switch for emergency stop of a system. If any of the manipulation members constituting the setting manipulation unit 30 is manipulated by the operator Op, a manipulation signal depending on the manipulation of the corresponding manipulation member is input from the setting manipulation unit 30 to the master processing circuit 27.

The master processing circuit 27 analyzes the manipulation signal from the manipulation input unit 11 and the manipulation signal from the setting manipulation unit 30. The master processing circuit 27 generates a control signal for controlling the medical manipulator system in accordance with a result of the analysis of the manipulation signal, and inputs the control signal to the slave control circuit 26.

The image-processing circuit 28 performs various image processing for displaying the image signal input from the endoscope 31, and generates image data for display on the display 29. The display 29 includes, for example, a liquid crystal display. The display 29 displays an image based on the image data generated by the image-processing circuit 28 in accordance with the image signal acquired through the endoscope 31.

In the medical manipulator system configured as described above, when the operator Op manipulates the manipulation input unit 11, the corresponding treatment tool 2 operates to correspond to a movement of the manipulation input unit 11. Thus, it is possible to perform a desired procedure on the patient P.

Next, a schematic configuration of the medical system 1 will be described.

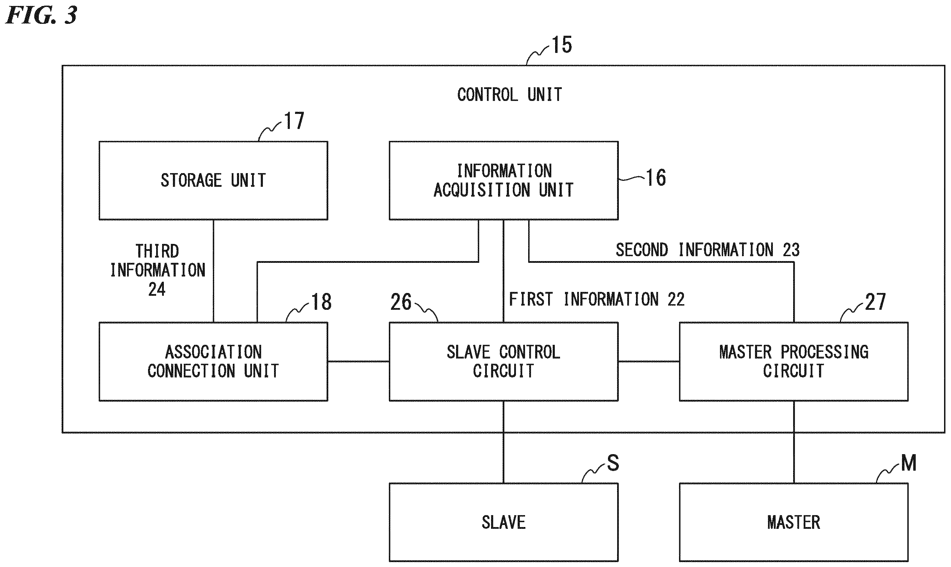

As illustrated in Figs. 1, 2 and 3, the medical system 1 includes the treatment tool 2, the driving unit 10, the manipulation input unit 11, and a control unit 15. In the present specification, mechanically connecting the treatment tool 2 to the control unit 15 through the driving unit 10, and mechanically connecting the manipulation input unit 11 to the control unit 15 are referred to as "attach". Further, connecting the treatment tool 2 to the manipulation input unit 11 via the control unit 15 and enabling the treatment tool 2 to be operated by the manipulation input unit 11 is referred to as "connect".

The treatment tool 2 includes a treatment portion 3 that performs a treatment with respect to a living body, such as grasping forceps or a cutting electrode, joint portions 4 that change a position and an orientation of the treatment portion 3, and an elongated insertion portion 5 for guiding the treatment portion 3 to a treatment target portion. Two treatment tools 2 are provided in the medical system 1, and are configured to be appropriately exchanged and attached to the driving unit 10 of the medical system 1. The treatment tool 2 may be a disposable treatment tool which is discarded in each surgery or each use.

A configuration of the treatment portion 3 may be appropriately selected depending on a procedure. The treatment portion 3 operates on the basis of a manipulation input in the manipulation input unit 11.

The joint portion 4 has, for example, a degree of freedom of one or more axes. The joint portion 4 operates on the basis of a manipulation input in the manipulation input unit 11.

The insertion portion 5 is a flexible tubular member into which a wire or the like (not illustrated) for causing the treatment portion 3 or the joint portion 4 to operate is inserted. In the insertion portion 5, a treatment tool connector 6 for attaching the treatment tool 2 to the driving unit 10 is provided.

The treatment tool connector 6 has first identification information (hereinafter referred to as "first information") capable of identifying a configuration of the treatment tool 2.

The first information in this embodiment is encoded and displayed as a bar code 7 on an outer surface of the treatment tool connector 6. The first information is not limited to the bar code 7 as long as the first information is provided at the treatment tool connector 6 in a format that can be read by the control unit 15. For example, the first information, may be stored in a storage device that can be read by the control unit 15.

Further, the first information may be an identifier capable of simply identifying the treatment tool 2, rather than information including a configuration of the treatment tool 2. In this case, a parameter or the like specific to the treatment tool 2 is stored in the control unit 15 in advance, and a parameter corresponding to the identifier of the treatment tool 2 may be loaded in the control unit 15.

The driving unit 10 includes a motor (not illustrated) for causing the treatment portion 3 and the joint portion 4 to operate. An operation of the motor of the driving unit 10 is controlled by the control unit 15. The motor of the driving unit 10 is attached to a wire or the like arranged in the insertion portion 5. The driving unit 10 according to this embodiment includes one or more motors corresponding to the treatment tool 2. An attachment form of the driving unit 10 to the wire or the like may be common to be attachable to a plurality of treatment tools 2 having different configurations.

The manipulation input unit 11 is configured to be manipulated in hand by the operator Op of the medical system 1. For the manipulation input unit 11, a manipulation input unit having a structure corresponding to a configuration of the treatment tool 2 or a user-friendly structure for the operator Op can be appropriately selected from a plurality of manipulation input units 11 having different configurations and attached to the control unit 15. Further, the manipulation input unit 11 may be disposable.

The manipulation input units 11A and is 11B include direction switches 12A and 12B and an opening and closing switch 19 for causing the treatment tools 2A and 2B to operate.

For example, in the manipulation input unit 11A assumed to manipulate a cutting electrode, the direction switch 12A serves as input means (input unit) for causing a joint portion 4A to operate.

For example, in the manipulation input unit 11B assumed to manipulate a grasping forceps, the direction switch 12B serves as input means (input unit) for causing the joint portion 4B to operate, and the opening and closing switch 19 serves as input means (input unit) for causing a treatment portion 3B (grasping forceps) to open and close.

The manipulation input unit 11 includes a manipulation input unit connector 13 for attaching the manipulation input unit 11 to the control unit 15, and second identification information (hereinafter referred to as "second information") capable of identifying a configuration of the manipulation input unit 11, in addition to each direction switch 12 and the opening and closing switch 19.

The second information in this embodiment is stored in a memory 14 (storage element) mounted inside the manipulation input unit 11. A form in which the second information is stored in the storage element is not limited as long as the second information, is provided at the manipulation input unit 11 in a format that can be read by the control unit 15. Further, a configuration of the memory element is not particularly limited, and the memory element may be a ROM, a RAM, an IC tag such as an RFID tag, a resistor, or any other electrical circuit. Further, the second information may also be displayed on the manipulation input unit 11 as an image encoded as a bar code 7 or the like.

Further, the second information may be an identifier capable of identifying the manipulation input unit 11. In this case, a parameter or the like specific to the manipulation input unit 11 may be stored in the control unit 15 in advance, and a parameter corresponding to the identifier of the manipulation input unit 11 may be loaded in the control unit 15.

The control unit 15 includes an information acquisition unit 16 that acquires first information of the treatment tool 2 and second information, of the manipulation input unit 11, a storage unit 17 in which information on the treatment tool 2 and the manipulation input unit 11 is stored, and an association connection unit 18 that associates the treatment tool 2 with the manipulation input unit 11 on the basis of the first information and the second information.

The information acquisition unit 16 includes an optical sensor (not illustrated) that reads the bar code 7 of the treatment tool 2, and a reading circuit (not illustrated) that reads the second information from the storage element of the manipulation input unit 11.

The storage unit 17 includes a database including a list of the treatment portion 3 and the joint portion 4 of each treatment tool 2 assumed to be attached to the medical system 1 according to this embodiment, and a list of each direction switch 12 and the opening and closing switch 19 of each manipulation input unit 11 assumed to be attached to the medical system 1 according to this embodiment. Further, the storage unit 17 includes a list (correspondence information) of the manipulation input units 11 capable of causing the treatment tool 2 to operate, for each treatment tool 2.

The association connection unit 18 identifies the treatment tool 2 and the manipulation input unit 11 attached to the control unit 15 on the basis of the first information and the second information acquired by the information acquisition unit 16. The association connection unit 18 generates the correspondence information table indicating a correspondence relationship between the treatment tool 2 and the manipulation input unit 11 by referring to each list stored in the database of the storage unit 17, and stores the correspondence information table in the storage unit 17. That is, the association connection unit 18 performs association between the treatment tool 2 and the manipulation input unit 11.

Specifically, when there is an associable combination stored in the correspondence information, (hereinafter referred to as "third information") among the treatment tool 2 and the manipulation input unit 11 attached to the control unit 15 on the basis of the first information and the second information, the association connection unit 18 first performs association in the combination stored in the third information and generates a correspondence information table. In this case, in the correspondence information table, the treatment portion 3A of the treatment tool 2A and the direction switch 12A of the manipulation input unit 11A have a correspondence relationship set in advance. Further, in this case, in the correspondence information table, the joint portion 4B of the treatment tool 2B and the direction switch 12B of the manipulation input unit 11B have a correspondence relationship set in advance, and the treatment portion 3B and the opening and closing switch 19 of the manipulation input unit 11B have a correspondence relationship set in advance. After the correspondence information table is generated, information indicating the direction switch 12 and the opening and closing switch 19 of the manipulation input unit 11 to be assigned to the treatment portion 3 and the joint portion 4 is presented to the operator Op by means which can be recognized by the operator Op.

An example of association among the first information, the second information, and the third information will be described in detail with reference to the drawings.

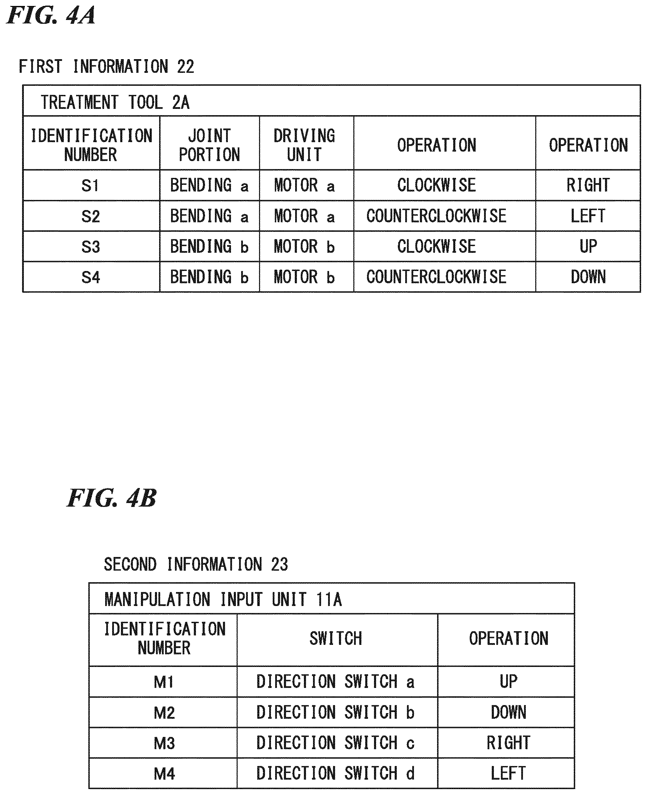

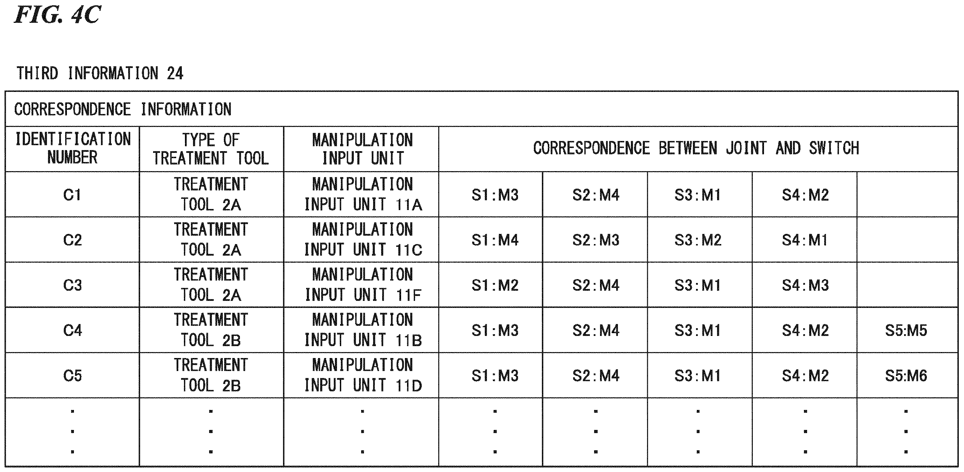

Fig. 4 is a conceptual diagram illustrating the first information 22, the second information 23, and the third information 24.

Information on the treatment tool 2 is stored in the first information 22 illustrated in Fig. 4A. In the first information 22, for example, a relationship between the joint portion 4A of the treatment tool 2A, an operation direction (for example, clockwise or counterclockwise) of the motor of the driving unit 10A, and an operation direction (for example, up, down, right, and left) of the joint portion 4A is stored in a table format. For example, in a top row of the table, an identification number S1 of the treatment tool 2A is information indicating that the joint portion 4A moves to the right when a motor a (not illustrated) of the driving unit 10A moves clockwise.

Information on the manipulation input unit 11 is stored in the second information 23 illustrated in Fig. 4B. For example, a relationship between the switch 12A (for example, a, b, c, and d) of the manipulation input unit 11A and a role (for example, up, down, right, and left) of the switch 12A is stored in a table format in the second information 23. For example, in a top row of the table, an identification number M1 of the manipulation input unit 11A is information indicating that the switch 12Aa indicates an upward manipulation instruction.

In the third information 24 illustrated in Fig. 4C, correspondence information of the treatment tool 2 and the manipulation input unit 11 is stored. In the third information 24, for example, information on correspondence among the treatment tool 2, the manipulation input unit 11, and the switch 12 is stored in a table format. For example, in a top row of the table, the treatment tool 2A and the manipulation input unit 11A are a corresponding combination, and a combination of the joint portion 4A of the treatment tool 2A and the switch 12A of the manipulation input unit 11A is information in which the respective identification numbers S1 and M3, S2 and M4, S3 and M1, S4 and M2 of the first information 22 and the second information 23 correspond to each other. Thus, for example, association between the identification number S3 in which an operation direction of the treatment tool 2A is up and the identification number M1 in which an operation direction of the manipulation input unit 11A is up is performed. As a result, when the switch 12Aa (up) of the manipulation input unit 11A is pressed, the control unit 15 receives information and moves a corresponding motor b of the driving unit 10A, which causes the joint portion 4A of the treatment tool 2A to operate, clockwise. An operation of the motor b is transferred to the joint portion 4A of the treatment tool 2A, and the joint portion 4A of the treatment tool 2A moves in an upward direction.

Correspondence information about the manipulation input unit 11 other than the manipulation input units 11A and 11B illustrated in Fig. 2 may be stored in the table of the third information 24 illustrated in Fig. 4C. For example, the manipulation input units 11C, 11D, and 11 F within items of the manipulation input unit 11 in the table of the third information 24 illustrated in Fig. 4C are information on the manipulation input unit 11 (not illustrated) other than the manipulation input units 11A and 11B illustrated in Fig. 2.

Thus, in this embodiment, by the association between the treatment portion 3 and the joint portion 4 of the treatment tool 2 and the direction switch 12 and the opening and closing switch 19 of the manipulation input unit 11 being performed, the association between the input to the manipulation input unit 11 and the operation of the treatment tool 2 is performed for the manipulation input unit 11 associated with the treatment tool 2 on the basis of a comparison among the first information 22, the second information 23, and the third information 24. After the association is performed, a correspondence relationship between each operation element, such as the treatment portion 3 and the joint portion 4, and each direction switch 12 and the opening and closing switch 19 may be changed individually, if necessary.

The association connection unit 18 according to this embodiment sets a priority or associable combination stored in the third information 24 to be higher than a combination which does not exist in the third information 24. The combination not stored in the third information 24 may be arbitrarily selected by the operator Op. In this case, using a method to be described below for setting of a combination not stored in the third information 24, the treatment tool 2 selected by the operator Op and the manipulation input unit 11 are first connected to each other. After setting or the combination not stored in the third information 24 is performed, the associable combination stored in the third information 24 is searched for, and the associable treatment tool 2 and the manipulation input unit 11 are connected to each other.

When there are the unconnected treatment tool 2 and the unconnected manipulation input unit 11 after the association based on the associable combination stored in the correspondence infomation ends or when a connection in the combination not stored in the third information 24 is performed by the selection of the operator Op, the connection is performed for the treatment tool 2 and the manipulation input unit 11 that can be connected, in accordance with the list stored in the storage unit 17.

Specifically, the association connection unit 18 reads a list of treatment portions 3 and joint portions 4 in the treatment tool 2 and sequentially assigns each direction switch 12 and the opening and closing switch 19 of the manipulation input unit 11 to the treatment portions 3 and the joint portions 4. For example, after each direction switch 12 and the opening and closing switch 19 of the manipulation input unit 11 are assigned to the treatment portion 3 and the joint portion 4 of the treatment tool 2 as a default value, information indicating the direction switch 12 and the opening and closing switch 19 of the manipulation input unit 11 assigned to the treatment portion 3 and the joint portion 4 is presented to the operator Op, and any change performed by the operator Op is received. In this case, only the direction switch 12 and the opening and closing switch 19 that can be assigned to the treatment tool 2 among each direction switch 12 and the opening and closing switch 19 of the manipulation input unit 11 are connected to the treatment tool 2, and the direction switch 12 and the opening and closing switch 19 that cannot be assigned are neglected.

Further, when there is the treatment portion 3 or the joint portion 4 in the treatment tool 2 that cannot be operated by the direction switch 12 and the opening and closing switch 19 of the manipulation input unit 11 attached to the control unit 15, the association connection unit 18 generates a warning indicating that a portion of the treatment tool 2 does not operate and causes the operator Op to select whether or not the connection is performed. In a case in which connection permission is input by the operator Op even when the portion of the treatment tool 2 does not operate, the association connection unit 18 connects only connectable portions in the treatment tool 2 and the manipulation input unit 11.

By all the treatment tools 2 and all the manipulation input units 11 attached to the control unit 15 being connected to have a correspondence relationship, the association of the treatment tool 2 and the manipulation input unit 11 by the association connection unit 18 ends. The treatment tool 2 and the manipulation input unit 11 with no correspondence relationship do not operate, or are manually detached from the control unit 15 in response to an output or the like from the control unit 15 that requests detachment.

When the treatment tool 2 and the manipulation input unit 11 are connected to each other by the association connection unit 18, the treatment portion 3 and the joint portion 4 are operable in response to the input to each direction switch 12 and the opening and closing switch 19 of the manipulation input unit 11, and treatment using the treatment tool 2 can be performed.

According to the method of setting the medical system 1 according to this embodiment, since the association between the treatment tool 2 and the manipulation input unit 11 attached to the control unit 15 is automatically performed, the Treatment tool 2 and the manipulation input units 11 are simply and easily associated with each other.

Further, since, even when the manipulation input unit 11 is not the manipulation input unit 11 corresponding to all operation elements of the treatment tool 2, the manipulation input unit 11 can be connected to the treatment tool 2 as the manipulation input unit 11 that causes a portion of the treatment tool 2 to operate, interconnection can be performed by various combinations of the treatment tool 2 and the manipulation input unit 11.

### (First modified example)

The third information 24 described in the above embodiment may be stored in the storage element of the manipulation input unit 11. Further, the treatment tool 2 may include a storage element, and the third information 24 described in the above embodiment may be stored in this storage element. In this modified example, the same effects as in the above embodiment are achieved.

### (Second modified example)

The control unit 15 described in the above embodiment may perform appropriate association when a plurality of manipulation input units 11 of the same type are attached to the control unit 15.

That is, when there are a plurality of manipulation input units 11 of the same type, the control unit 15 associates the first detected manipulation input unit 11 with the first detected treatment tool 2, and sequentially associates the other manipulation input units 11 with the other treatment tools 2. The control unit 15 can determine a detection order for the treatment tool 2 and the manipulation input unit 11 by prioritizing the treatment tool connector 6 and the manipulation input unit connector 13.

For example, connectors 20A and 20B (see Fig. 2) provided at the control unit 15 to correspond to the driving unit 10 to which the first treatment tool connector 6A and the second treatment tool connector 6B can be respectively attached may have a priority in the control unit 15. In this case, the control unit 15 first accesses the connector 20A in order to detect whether the treatment tool 2 corresponding to the first treatment tool connector 6A has been attached. Subsequently, the control unit 15 accesses the connector 20B in order to detect whether the treatment tool 2 corresponding to the second treatment tool connector 6B has been attached.

Further, for example, the connectors 21A and 21B (see Fig. 2), which are provided at the control unit 15 such that the first manipulation input unit connector 13A and the second manipulation input unit connector 13B can be respectively attached to the connectors 21A and 21B, have a priority in the control unit 15. In this case, the control unit 15 first accesses the connector 21A in order to detect whether the manipulation input unit 11 corresponding to the first manipulation input unit connector 13A has been attached. Subsequently, the control unit 15 accesses the connector 21B for detecting whether the manipulation input unit 11 corresponding to the second manipulation input unit connector 13B has been attached.

Subsequently, the control unit 15 performs association corresponding to an associable combination stored in the third information, 24 described in the above embodiment. In this case, when the manipulation input unit 11 capable of operating the treatment tool 2 corresponding to the first treatment tool connector 6A is simultaneously attached to the connector 21 A and the connector 21B, the manipulation input unit 11 corresponding to the first manipulation input unit connector 13A attached to the connector 21A having a higher priority is connected to the treatment tool 2 which is a connection target prior to the manipulation input unit 11 corresponding to the second manipulation input unit connector 13B attached to the connector 21B having a lower priority than the connector 21 A. The connectors 20A, 20B, 21A, and 21B are processed in order in accordance with their priorities.

Further, in this modified example, even when the combination is not an associable combination stored in the correspondence information, only the direction switch 12 and the opening and closing switch 19 that can be assigned to the treatment tool 2 among each direction switch 12 and the opening and closing switch 19 of the manipulation input unit 11 are connected to the treatment tool 2, and the direction switch 12 and the opening and closing switch 19 that cannot be assigned are neglected. Thus, some or all of the treatment tools 2 can be operated by the manipulation input unit 11.

### (Third modified example)

In this modified example, after the association between the treatment tool 2 and the manipulation input unit 11 is completed, a correspondence relationship between the treatment portion 3 and the joint portion 4, and each direction switch 12 and the opening and closing switch 19 of the manipulation input unit 11 can be changed.

Specifically, the control unit 15 presents a list of the direction switch 12 and the opening and closing switch 19 which can cause the treatment portion 3 to operate among each direction switch 12 and the opening and closing switch 19 of the manipulation input unit 11 to the operator Op. Further, the control unit 15 presents a list of the direction switch 12 and the opening and closing switch 19 which can cause the joint portion 4 to operate among each direction switch 12 and the opening and closing switch 19 of the manipulation input unit 11 to the operator Op. The control unit 15 receives an input of the assignment of each direction switch 12 and the opening and closing switch 19 to the treatment portion 3 and the joint portion 4. The control unit 15 may have a plurality of items of preset data, in which a correspondence relationship between each direction switch 12 and the opening and closing switch 19, and the operation elements such as the treatment portion 3 and the joint portion 4 in the treatment tool 2 is defined, in the storage unit 17. The operator Op may be caused to select one correspondence relationship from the preset data.

With this configuration, the same effects as the above embodiment can also be achieved. In this modified example, an easy-to-use setting can be easily selected for the operator Op.

Next, a specific example of a method of setting a medical system according to this embodiment will be described in detail with reference to flowcharts.

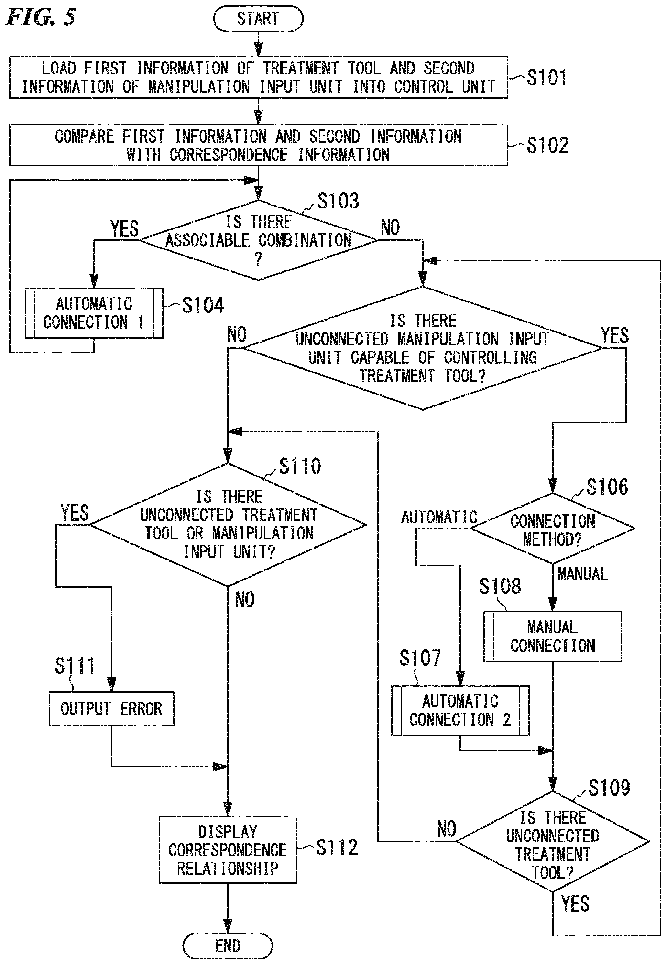

Fig. 5 to 11 are flowchart illustrating a method of setting the medical system 1. Reference numerals of the components of the medical system 1 in the following description based on the flowcharts are illustrated in Figs. 1, 2 and 3.

Step S101 illustrated in Fig. 5 is started when the treatment tool 2 and the manipulation input unit 11 are attached to the control unit 15.

In step S101, the control unit 15 loads the first information 22 (which is, for example, encoded in the bar code 7) of the treatment tool 2 and the second information, 23 (which is, for example, stored in the memory 14) of the manipulation input unit 11 into the storage unit 17.

Step S101 ends and the process proceeds to step S102.

Step S102 is a step of comparing the first information 22, the second information 23, and the third information 24 (which is, for example, stored in the storage unit 17) with one another.

In step S102, the storage unit 17 refers to a list (third information 24) of manipulation input units 11 (which can cause the treatment tool 2 to operate in order to determine whether or not the first information 22 of the treatment tool 2 and the second information 23 of the manipulation input unit 11 are listed in the third information 24.

Step S102 ends and the process proceeds to step S103.

Step S103 is a step of determining whether or not there is an associable combination on the basis of the first information 22, the second information 23, and the third information 24.

In step S103, when there is the associable combination, the process proceeds to step S104 ("Automatic connection 1") to be described below, and when there is no associable combination, the process proceeds to step S105.

Step S104 is a step of connecting the treatment tool 2 and the manipulation input unit 11 that are an associable combination to each other and associating the direction switch 12 and the opening and closing switch 19 of the manipulation input unit 11 with the operation elements such as the treatment portion 3 and the joint portion 4. A flow of a specific process in step S104 will be described below.

Step S104 ends and the process proceeds to step S103. That is, step S104 is repeated until there is no associable combination.

Step S105 is started when it is determined in step S103 that there is no associable combination.

In step S105, it is detected whether or not there is an unconnected manipulation input unit 11 capable of controlling the treatment tool 2. That is, when there is a manipulation input unit 11 that is attached to the control unit 15 but is not connected to the treatment tool 2, the process proceeds to step S106, and when there is no manipulation input unit 11 that is attached to the control unit 5 but is not connected to the treatment tool 2, the process proceeds to step S110.

Step S106 is a step of determining a method of connecting the manipulation input unit 11 that is attached to the control unit 15 but is not connected to the treatment tool 2, to the treatment tool 2.

In step S106, any one of step S107 ("Auto Connection 2") to be described below and step S108 ("Manual Connection") to be described below is executed on the basis of the input from the operator Op or on the basis of whether or not automatic connection is possible.

Step S107 is a step of connecting the treatment tool 2 to the manipulation input unit 11 that is not an associable combination but can control the treatment tool 2, and performing an association of the direction switch 12 and the opening and closing switch 19 in a possible range. Details of a flow of a process in step S107 will be described below.

Step S107 ends and the process proceeds to step S109.

Step S108 is a step of connecting the treatment tool 2 to the manipulation input unit 11 that is not an associable combination but can control the treatment tool 2, and performing an association of the direction switch 12 and the opening and closing switch 19 on the basis of an input of association from the operator Op. Details of a flow of a process in step S108 will be described below.

Step S308 ends and the process proceeds to step S109.

Step S109 is a step of determining whether or not there is an unconnected treatment tool 2.

In step S109, when there is an unconnected treatment tool 2, the process proceeds to step S105, and when there is no unconnected treatment tool 2, the process proceeds to step S110. That is, each step from step S105 to step S108 described above is repeated until the unconnected treatment tool 2 is not detected.

Step S110 is a step of determining whether or not there is an unconnected treatment tool 2 or an unconnected manipulation input unit 11.

In step S110, when the treatment tool 2 or the manipulation input unit 11 is in an unconnected state, the process proceeds to step S111 to indicate an error message to the operator Op. When the treatment tool 2 or the manipulation input unit 11 is not in an unconnected state, all of the treatment tools 2 connected to the control unit 15 can be controlled and there is no extra manipulation input unit 11. Accordingly, the process proceeds to step S112.

Step S111 is a step of presenting a treatment tool 2 which cannot be controlled or an extra manipulation input unit 11 to the operator Op using an error message when there is the treatment tool 2 which cannot be controlled or when there is the extra manipulation input unit 11.

In step S111, the error message is presented and the operator Op is requested to perform work such as continuing with the setting as it is, invalidating or detaching the treatment tool 2 that cannot be controlled, or invalidating or detaching the extra manipulation input unit 11.

Step S111 ends and the process proceeds to step S112.

Step S112 is a step of presenting the operation element of the treatment tool 2 to which each direction switch 12 and the opening and closing switch 19 of the manipulation input unit 11 correspond, to the operator Op. In step S112, an input for changing a correspondence relationship between the direction switch 12 and the opening and closing switch 19, and the operation element may be received.

Step S112 ends, the setting of the medical system 1 ends, and the medical system 1 becomes available.

Next, a flow of step S104 ("Automatic connection 1") described above will be described.

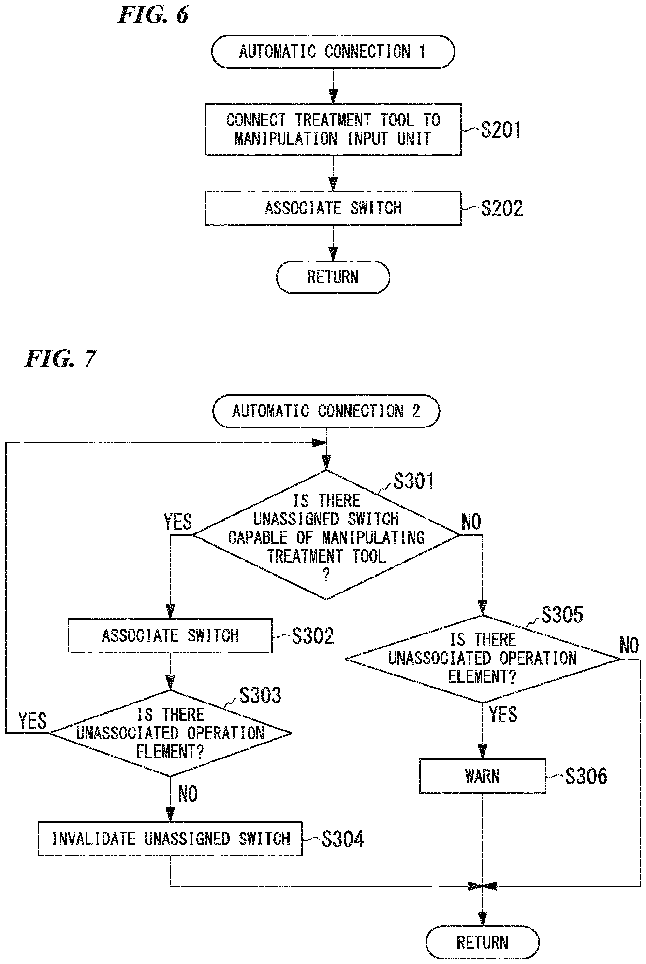

Step S201 illustrated in Fig. 6 is a step that is branched from step S103 described above and started, and in which the associate treatment tool 2 and manipulation input unit 11 are connected to each other. By the treatment tool 2 being connected to the manipulation input unit 11, a one-to-one correspondence occurs between the treatment tool 2 and the manipulation input unit 11.

Step S201 ends and the process proceeds to step S202.

In Step S202, for the treatment tool 2 and the manipulation input unit 11 between which the one-to-one correspondence relationship is set in step S201 described above, the direction switch 12 and opening and closing switch 19 of the manipulation input unit 11 are associated with the operation elements such as the treatment portion 3 and the joint portion 4 of the treatment tool 2. As an example, in step S202, association between on-off of the direction switch 12 and the opening and closing switch 19, and on-off of the motor of the driving unit 10 or the operation direction is performed, and a result of the association is stored in the storage unit 17.

The association in step S202 is performed based on a default correspondence relationship stored in the storage unit 17 in advance on the basis of, for example, ease of manipulation. The correspondence relationship may be changed from the correspondence relationship defined in step S112 described above depending on an input of the operator Op.

Step S202 ends and the process proceeds to step S103 described above.

Next, a flow of "Auto connection 2" described above will be described.

Step S301 illustrated in Fig. 7 is a step that is branched from step S106 described above and started.

In step S301, one manipulation input unit 11 capable of controlling one treatment tool 2 on which the association has not been performed in step S104 described above is selected, and it is determined whether or not there is, in this manipulation input unit 11, the unassigned direction switch 12 and the unassigned opening and closing switch 19 capable of operating the treatment tool 2. A condition in which there are no unassigned direction switch 12 and unassigned opening and closing switch 19 capable of operating the treatment tool 2 has been excluded through the determination in step S105 in this embodiment. When there is the unassigned direction switch 12 capable of manipulating the treatment tool 2, the process proceeds to step S302, and when there are no unassigned direction switch 12 and unassigned opening and closing switch 19 capable of operating the treatment tool 2, the process proceeds to step S305.

Step S302 is a step of associating the operation element of the treatment tool 2 with the unassigned direction switch 12 and the unassigned opening and closing switch 19.

In step S302, a one-to-one correspondence relationship between the direction switch 12 and the opening and closing switch 19, and the operation elements of the treatment tool 2 is set on the basis of a predetermined priority or preset information.

Step S302 ends and the process proceeds to step S303.

Step S303 is a step of determining whether or not the respective operation elements of the treatment tool 2 are associated with each direction switch 12 and the opening and closing switch 19 of the manipulation input unit 11.

In step S303, when all the operation elements of the treatment tool 2 are associated with the direction switch 12 and the opening and closing switch 19, the treatment tool 2 can be operated in a perfect state. Accordingly, further association is unnecessary. Further, in step S303, when there is an operation element that is not associated with the direction switch 12 and the opening and closing switch 19, steps S301 and S302 described above are repeated to attempt the assignment of the direction switch 12 and the opening and closing switch 19 to all the operation elements.

When there is no operation element that is not associated with the direction switch 12 and the opening and closing switch 19, the process proceeds to step S304, and when there is an operation element that is not associated with the direction switch 12 and the opening and closing switch 19, the process proceeds to step S301.

Step S304 is a step of invalidating the unassigned direction switch 12 and the unassigned opening and closing switch 19.

In step S304, since switches other than the direction switch 12 and the opening and closing switch 19 of which the association has been performed are not necessary to operate the treatment tool 2, the unassigned direction switch 12 and the unassigned opening and closing switch 19 are all invalidated so that an input to the unassigned direction switch 12 and the unassigned opening and closing switch 19 is neglected.

Step S304 ends and the process proceeds to step S109.

Step S305 is a step of determining whether or not there is an operation element that is not associated under a condition in which there are no unassigned direction switch 12 and unassigned opening and closing switch 19 capable of manipulating the treatment tool 2. That is, step S305 is a step of determining whether the treatment tool 2 fully operates or a portion of the treatment tool 2 does not operate.

In step S305, when it is determined that there is an operation element that is not associated, the process proceeds to step S306, and when it is determined that there is no operation element that is not associated, the process proceeds to step S109.

Step S306 is a step of presenting a warning that a portion of the treatment tool 2 does not operate to the operator Op.

In step S306, an input as to whether or not a setting of a state in which the portion of the treatment tool 2 does not operate is continued may be received.

Step S306 ends and the process proceeds to step S109.

Next, a flow of the "manual connection" will be described.

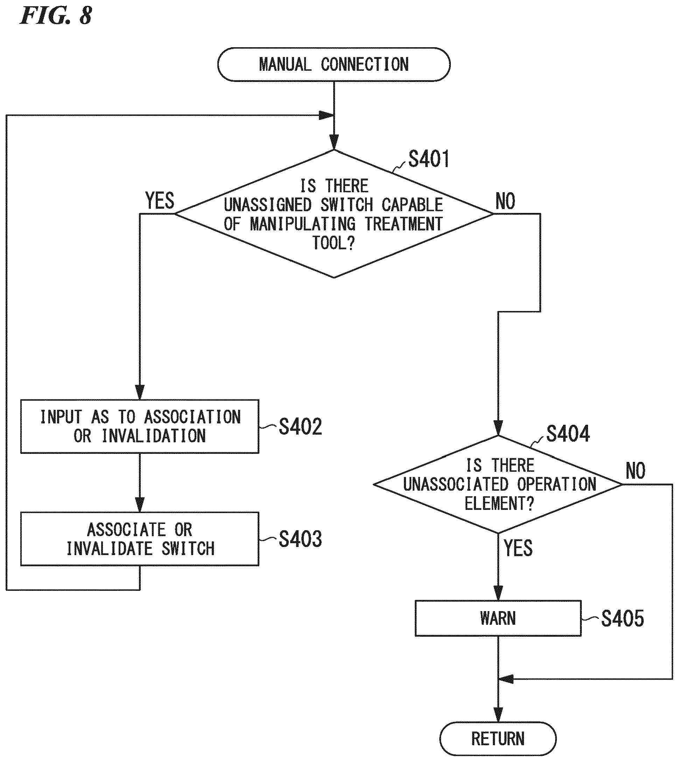

Step S401 illustrated in Fig. 8 is a step which is branched from step S106 described above and started.

In step S401, one manipulation input unit 11 capable of controlling one treatment tool 2 on which the association has not been performed in step S104 described above is selected, and it is determined whether or not there is, in this manipulation input unit 11, the unassigned direction switch 12 and the unassigned opening and closing switch 19 capable of manipulating the treatment tool 2. A condition in which there are no unassigned direction switch 12 and unassigned opening and closing switch 19 capable of manipulating the treatment tool 2 has been excluded through the determination in step S105 described above in this embodiment. When there are the unassigned direction switch 12 and the unassigned opening and closing switch 19 capable of manipulating the treatment tool 2, the process proceeds to step S402, and when there are no unassigned direction switch 12 and unassigned opening and closing switch 19 capable of manipulating the treatment tool 2, the process proceeds to step S404.

Step S402 is a step of receiving an input from the operator Op with respect to the association of the direction switch 12 and the opening and closing switch 19 with the operation elements of the treatment tool 2 or invalidation of the direction switch 12 and the opening and closing switch 19.

In step S402, an input as to whether one of the unassigned direction switch 12 and the unassigned opening and closing switch 19 is associated with any operation element of the treatment tool 2 or is invalidated so that the one does not use is performed by the operator Op.

Step S402 ends and the process proceeds to step S403.

Step S403 is a step of associating or invalidating the direction switch 12 and the opening and closing switch 19 in accordance with the input in step S402 described above.

When the association or the invalidation is performed in step S403, step S403 ends and the process proceeds to step S401. That is, steps S401 to S403 are repeated until the association or the invalidation is performed on all of unassigned direction switches 12 and unassigned opening and closing switches 19 by the operator Op.

Step S404 is a step of determining whether or not there is an operation element that has not been associated under a condition in which there is no unassigned switch capable of manipulating the treatment tool 2.

That is, step S404 is a step of determining whether the treatment tool 2 fully operates or a portion of the treatment tool 2 does not operate.

In step S404, when it is determined that there is an operation element that has not been associated, the process proceeds to step S405, and when it is determined that there is no operation element that has not been associated, the process proceeds to step S109.

Step S405 is a step of presenting a warning that a portion of the treatment tool 2 does not operate to the operator Op.

In step S405, an input as to whether or not a setting of a state in which the portion of the treatment tool 2 does not operate continues may be received.

Step S405 ends and the process proceeds to step S109.

Next, a method of setting the medical system 1 in which the manipulation input unit 11 of the same type is assumed to be attached to the medical system 1 will be described.

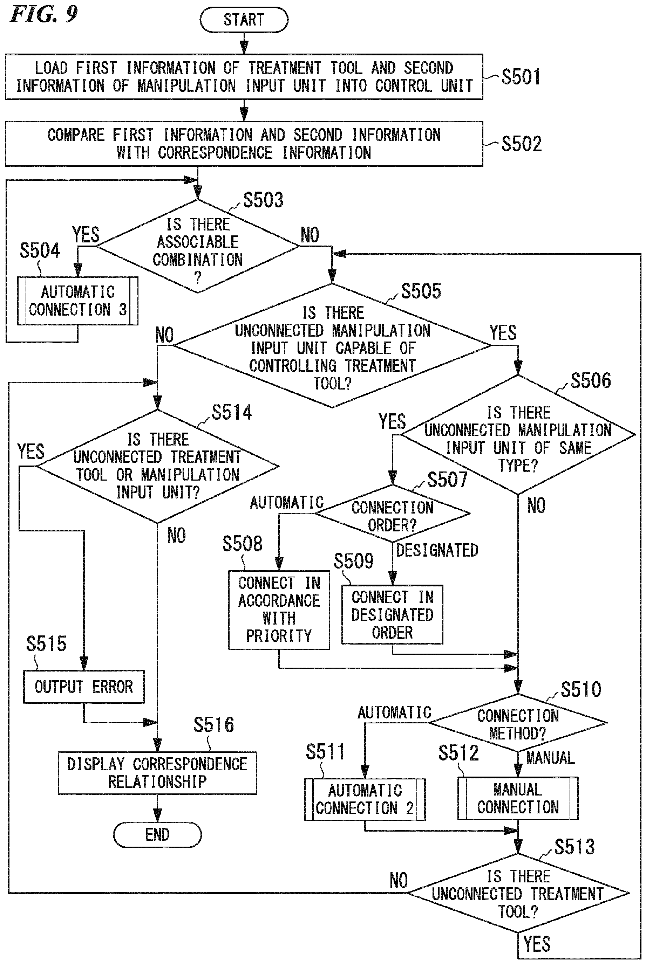

Step S501 illustrated in Fig. 9 is a step of loading the first information 22 of the treatment tool 2 and the second information 23 of the manipulation input unit 11 into the control unit 15.

In step S501, for example, when there are a plurality of manipulation input units 11 of the same type, the plurality of manipulation input units 11 are distinguished and recognized using a method such as impacting a unique identifier in an order of detection of the manipulation input units 11.

Step S501 ends and the process proceeds to step S502.

Step S502 is a step of comparing the first information 22 and the second information 23 with the third information 24 stored in the storage unit 17.

Step S502 is the same as step S102 described above.

Step S502 ends and the process proceeds to step S503.

Step S503 is a step of performing the same determination as in step S103 described above. When there is an associable combination in step S503, the process proceeds to step S504 ("Automatic connection 3") to be described below, and when there is no associable combination, the process proceeds to step S505.

Step S504 is a step of performing a connection in consideration of a probability of the manipulation input units 11 of the same type being connected to the control unit 15 of the medical system 1.

Details of the flow of the connection in step S504 will be described below.

Step S504 ends and the process proceeds to step S503. That is, steps S503 and S504 are repeated until there is no associable combination.

Step S505 is a step of detecting the manipulation input unit 11 capable of controlling the treatment tool 2 that is not listed in the correspondence information as an associable combination, similarly to step S105 as described above. In step S505, when the manipulation input unit 11 capable of controlling the treatment tool 2 is detected, the process proceeds to step S506, and when the manipulation input unit 11 capable of controlling the treatment tool 2 is not detected, the process proceeds to step S514.

Step S506 is a step of determining whether or not, when there are a plurality of manipulation input units 11 that have been not associated, there are manipulation input units 11 of the same type among the plurality of manipulation input units 11.

In step S506, when there are manipulation input units 11 of the same type, the process proceeds to step S507, and when there are no manipulation input units 11 of the same type, the process proceeds to step S510.

Step S507 is a step of selecting which of the manipulation input units 11 of the same type attempts to connect to the treatment tool 2.

In step S507, since the plurality of manipulation input units 11 of the same type are distinguished in step S501 described above, an indication that the manipulation input units 11 are automatically connected to the treatment tool 2 in order in accordance with a predetermined priority is presented to the operator Op. In step S507, for the plurality of manipulation input units 11 of the same type, the operator Op is allowed to designate a specific manipulation input unit 11. When the treatment tool 2 is automatically connected, the process proceeds to step S508, and when the manipulation input unit 11 is designated by the operator Op, the process proceeds to step S509.

Step S508 is a step of setting a mode in which the plurality of manipulation input units 11 of the same type are connected to the treatment tool 2 in accordance with a predetermined priority.

In step S508, for example, an order for connecting the plurality of manipulation input units 11 of the same type to the plurality of treatment tools 2 in accordance with a predetermined priority is stored in the storage unit 17. Thus, when step S507 is executed again via step S513 from step S510 to be described below, an indication that the connection to the treatment tool 2 is performed in accordance with the order set in step S508 is presented to the operator Op.

Step S508 ends and the process proceeds to step S510.

Step S509 is a step of setting a mode in which a connection of the manipulation input unit 11 designated in step S507 to the treatment tool 2 is attempted.

In step S509, when step S507 is executed again via step S513 from step S510 to be described below, designation of the manipulation input unit 11 to be next connected to the treatment tool 2 or an option of an automatic connection is presented to the operator Op.

Step S509 ends and the process proceeds to step S510.

Steps S510, S511, S512, S513, S514, S515, and S516 are steps corresponding to steps S106, S107, S108, S109, S110, S111, and S112 described above, respectively.

In steps S510, S511, S512, S513, S514, S515, and S516, connection ("Auto connection 2" or "Manual connection" described above) of the first manipulation input unit in the order set in step S508 described above or the manipulation input unit 11 designated in step S509 described above to the treatment tool 2 is performed.

Next, a flow of step S504 "Automatic connection 3" described above will be described.

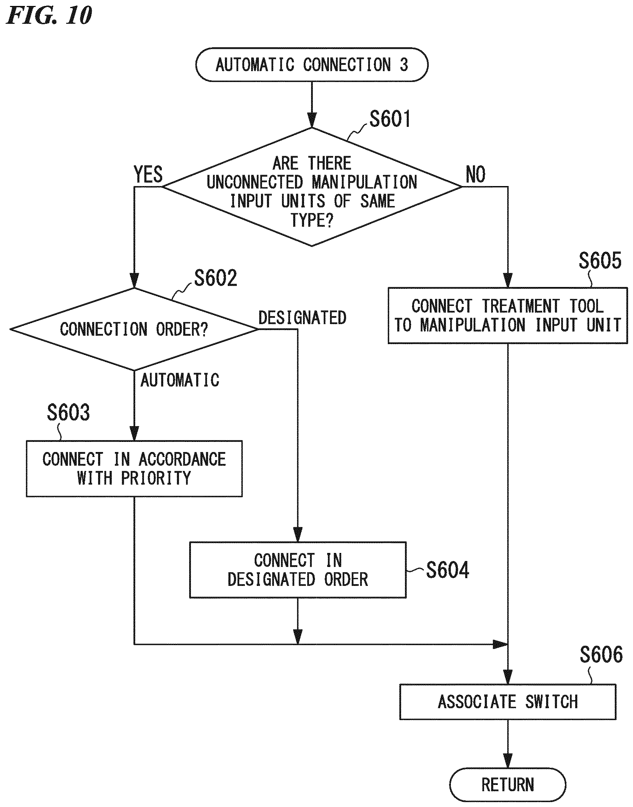

Step S601 illustrated in Fig. 10 is a step that is branched from step S503 and started when it is determined in step S503 that there is an associable manipulation input unit 11.

In step S601, it is determined whether or not, when there are a plurality of associable manipulation input units 11, there are manipulation input units 11 of the same types among the plurality of associable manipulation input units 11. In step S601, when there is a manipulation input unit 11 of the same type, the process proceeds to step S602, and when there is no manipulation input unit 11 of the same type, the process proceeds to step S605.

Step S602 is a step of selecting which of the manipulation input units 11 of the same type attempts to connect to the treatment tool 2.

In step S602, since the plurality of manipulation input units 11 of the same type are distinguished in step S501 described above, an indication that the manipulation input units 11 are automatically connected to the treatment tool 2 in order in accordance with a predetermined priority is presented to the operator Op. In step S602, for the plurality of manipulation input units 11 of the same type, the operator Op is allowed to designate a specific manipulation input unit 11. When the treatment tool 2 is automatically connected, the process proceeds to step S603, and when the manipulation input unit 11 is designated by the operator Op, the process proceeds to step S604.

Step S603 is a step of setting a mode in which the plurality of manipulation input units 11 of the same type are connected to the treatment tool 2 in accordance with a predetermined priority.

In step S603, for example, an order for connecting the plurality of manipulation input units 11 of the same type to the plurality of treatment tools 2 in accordance with a predetermined priority is stored in the storage unit 17. Thus, when step S602 is executed again via step S503 from step S606 to be described below, an indication that the connection to the treatment tool 2 is performed in accordance with the order set in step S603 is presented to the operator Op.

Step S603 ends and the process proceeds to step S606.

Step S604 is a step of setting a mode in which a connection of the manipulation input unit 11 designated in step S602 to the treatment tool 2 is attempted.

In step S604, when step S602 is executed again via step S503 from S606 to be described below, designation of the manipulation input unit 11 to be next connected to the treatment tool 2 or an option of an automatic connection is presented to the operator Op.

Step S604 ends and the process proceeds to step S606.

Step S605 is a step of connecting the associable manipulation input unit 11 to the treatment tool 2 when there is no unconnected manipulation input unit 11 of the same type under a condition in which there is the associable manipulation input unit 11.

When the manipulation input unit 11 is connected to the treatment tool 2 in step S605, step S605 ends and the process proceeds to step S606.

Step S606 is a step of associating each direction switch 12 and the opening and closing switch 19 of the manipulation input unit 11 to the operation elements of the treatment tool 2, similar to step S202 as described above.

Step S606 ends and the process proceeds to step S503.

Next, a case in which there are a plurality of treatment tools of the same type of step S504 "Automatic connection 3" described above will be described.

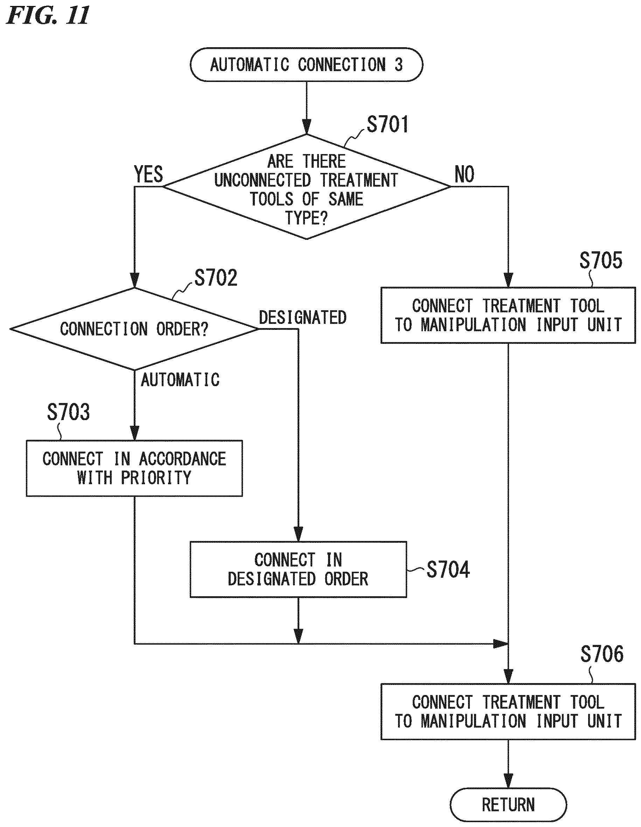

Step S701 illustrated in Fig. 11 is a step that is branched from step S503 and started when it is determined in step S503 that there is the associable manipulation input unit 11.

In step S701, it is determined whether or not, when there are a plurality of associable treatment tools 2, there are treatment tools 2 of the same type among the plurality of treatment tools 2. In step S701, when there are treatment tools 2 of the same type, the process proceeds to step S702, and when there no treatment tools 2 of the same type, the process proceeds to step S705.

Step S702 is a step of selecting which of the treatment tools 2 of the same type attempts to connect to the manipulation input unit 11.

In step S702, since the plurality of treatment tools 2 of the same type are distinguished in step S501 described above, an indication that the treatment tools 2 are automatically connected to the manipulation input unit 11 in order in accordance with a predetermined priority is presented to the operator Op. In step S702, for the plurality of treatment tools 2 of the same type, the operator Op is allowed to designate a specific treatment tool 2. When the manipulation input unit 11 is automatically connected, the process proceeds to step S703, and when there is designation of the treatment tool 2 performed by the operator Op, the process proceeds to step S704.

Step S703 is a step of setting a mode in which a plurality of treatment tools 2 of the same type are connected to the manipulation input unit 11 in accordance with a predetermined priority.

In step S703, for example, an order for connection of the plurality of treatment tools 2 of the same type to a plurality of manipulation input units 11 in accordance with a predetermined priority is stored in the storage unit 17. Thus, when step 702 is executed again via step 503 from step S706 to be described below, an indication that the connection to the manipulation input unit 11 is performed in the order set in step S703 is presented to the operator Op.

Step S703 ends and the process proceeds to step S706.

Step S704 is a step of setting a mode in which connection of the treatment tool 2 designated in step S702 to the manipulation input unit 11 is attempted.

In step S704, when step S702 is executed again via step 503 from step S706 to be described below, designation of the treatment tool 2 to be next connected to the manipulation input unit 11 or an option of an automatic connection is presented to the operator Op.

Step S704 ends and the process proceeds to step S706.

Step S705 is a step of connecting the associable treatment tool 2 to the manipulation input unit 11 when there is no unconnected treatment tool 2 of the same type under a condition in which there is the associable treatment tool 2.

When the treatment tool 2 is connected to the manipulation input unit 11 in step S705, step S705 ends and the process proceeds to step S706.

Step S706 is a step of associating each direction switch 12 and the opening and closing switch 19 of the manipulation input unit 11 with the operation elements of the treatment tool 2, similar to step S202 described above.

Step S706 ends and the process proceeds to step S503.

Although the embodiments of the present invention have been described in detail with reference to the drawings, specific configurations are not limited to the embodiments, and design changes or the like without departing from the scope of the present invention are also included.

Further, the components shown in the embodiments and the modified examples described above can be combined and configured appropriately.

The present invention is not limited by the foregoing description, but is only limited by the appended claims.

### Industrial Applicability

According to the method of setting a medical system according to the embodiment, it is possible to simply and easily associate the treatment tool with the manipulation input unit.

### Reference Signs List

- 1:: Medical system
- 2:: Treatment tool
- 3:: Treatment portion (operation element)
- 4:: Joint portion (operation element)
- 5:: Insertion portion
- 6:: Treatment tool connector
- 7:: Bar code
- 10:: Driving unit
- 11:: Manipulation input unit
- 12:: Direction switch
- 13:: Manipulation input unit connector
- 14:: Memory (storage element)
- 15:: Control unit
- 16:: Information acquisition unit
- 17:: Storage unit
- 18:: Association connection unit
- 19:: Opening and closing switch
- 20A, 20B:: Connector
- 21A, 21B:: Connector
- 22:: First information
- 23:: Second information
- 24:: Third information
- 25:: Surgical table
- 26:: Slave control circuit
- 27:: Master processing circuit
- 28:: Image-processing circuit
- 29:: Display
- 30:: Setting manipulation unit
- 31:: Endoscope

## Claims

1. A method of setting a medical system in which at least one treatment tool and at least one manipulation input unit for manipulating the treatment tool are connectable to each other, the method comprising:
a step of acquiring first information capable of identifying the treatment tool and second information capable of identifying the manipulation input unit;
a step of associating the treatment tool with the manipulation input unit on the basis of a comparison between the first information and the second information; and
a step of associating an input to the manipulation input unit with an operation of the treatment tool with respect to the manipulation input unit associated with the treatment tool on the basis of the comparison between the first information and the second information.

2. The method of setting the medical system according to claim 1, wherein the first information is acquired from image information displayed on the treatment tool.

3. The method of setting the medical system according to claim 1, wherein the second information is acquired from a storage element mounted on the manipulation input unit.

4. The method of setting the medical system according to claim 1, wherein third information indicating a correspondence relationship between the input to the manipulation input unit and the operation of the treatment tool is acquired from a storage element mounted on the manipulation input unit.

5. The method of setting the medical system according to claim 4, wherein a plurality of operation elements in the treatment tool are individually settable for a plurality of input units in the manipulation input unit.
